# EUROPEAN PATENT SPECIFICATION

(11) **EP 2 210 871 B1**
(45) Date of publication and mention of the grant of the patent: **18.03.2015**
(21) Application number: 09168191.6
(22) Date of filing: 09.07.2002
(51) Int. Cl.: C07C 209/36, C07C 209/68, C07C 211/56

(54) **Process for preparing 4-aminodiphenylamines**
Verfahren zur Herstellung von 4-Aminodiphenylaminen
Procédé de préparation de 4-aminodiphénylamine

(30) Priority: 23.07.2001 US 911058; 10.05.2002 US 143478
(43) Date of publication of application: 28.07.2010
(62) Divisional of application: 02742396.1
(73) Proprietor: FLEXSYS AMERICA L.P., Akron, Ohio 44333-0444 (US)
(72) Inventor: Triplett II, Ralph, Dale, Wadsworth, OH 44281 (US); Rains, Roger, Keranen, Richfield, OH 44286 (US)
(74) Representative: Beetz, Tom

(56) References cited:
- EP-A- 0 566 783
- WO-A-00/35853
- US-A- 5 117 063
- US-A- 5 453 541
- US-A- 5 608 111
- US-A- 5 739 403
- STERN, M.K. ET AL.: "Direct coupling of aniline and nitrobenzene: A new example of nucleophilic aromatic substitution for hydrogen" J. AM. CHEM. SOC., vol. 114, no. 23, 1992, pages 9237-9238, XP002216864

## Description

### BACKGROUND OF THE INVENTION

### 1. Field of the Invention

The present invention relates to a process for preparing 4-aminodiphenylamines intermediates.

### 2. Related Art

4-Aminodiphenylamines are widely used as intermediates in the manufacture of alkylated derivatives having utility as antiozonants and antioxidants, as stabilizers for monomers and polymers, and in various specialty applications. For example, reductive alkylation of 4-aminodiphenylamine (4-ADPA) with methylisobutyl ketone provides N-(1,3-dimethylbutyl)-N'-phenyl-p-phenylene-diamine, which is a useful antiozonant for the protection of various rubber products.

4-Aminodiphenylamine derivatives can be prepared in various ways. An attractive synthesis is the reaction of an optionally substituted aniline with an optionally substituted nitrobenzene in the presence of a base, as disclosed, for example, in U.S. 5,608,111 (to Stern et al.) and U.S. 5,739,403 (to Reinartz et al.).

U.S. 5,608,111 describes a process for the preparation of an optionally substituted 4-ADPA wherein in a first step optionally substituted aniline and optionally substituted nitrobenzene are reacted (coupled) in the presence of a base. In working examples, aniline and nitrobenzene are reacted in the presence of tetramethylammonium hydroxide as the base, and water and aniline are azeotropically removed during the coupling reaction.

International publication WO 00/35853 discloses a method of preparation of intermediates of 4-aminodiphenylamine by the reaction of aniline with nitrobenzene in a liquid medium where the reaction system consists of a solution of salts of true zwitterions with hydroxides. A combination of potassium hydroxide and betaine hydrate is exemplified. The reaction may take place in the presence of free oxygen.

EP publication 566 783 describes a method of manufacture of 4- nitrodiphenylamine by the reaction of nitrobenzene with aniline in the medium of a polar aprotic solvent in a strongly alkaline reaction system. A phase transfer catalyst such as tetrabutylammonium hydrogen sulfate is employed. This reference requires that the reaction be carried out in an oxygen-free atmosphere in order to prevent undesirable side reactions caused by oxidation.

US Patent No. 5,117,063 and International publication WO 01/14312 disclose processes for preparing 4-nitrodiphenylamine and 4-nitrosodiphenylamine, using an inorganic base with crown ether, a phase transfer catalyst.

US Patent No. 5,453,541 teaches that an external desiccant, such as anhydrous sodium sulfate, may be used to absorb excess water in an anaerobic or aerobic process for producing one or more 4-ADPA intermediates in which substituted aniline derivatives and nitrobenzene are brought into reactive contact.

The objective of the present invention is to provide a superior method for producing one or more 4-ADPA intermediates by reacting aniline and nitrobenzene in the presence of an organic base and an inorganic salt or a metal organic salt.

### SUMMARY OF THE INTENTION

The present invention is a method of producing 4-aminodiphenylamine or substituted derivatives thereof comprising the steps of:
(a) bringing an aniline or aniline derivative selected from the group consisting of formanilide, phenylurea, carbanilide and thiocarbanilide, or from the group consisting of 2-methoxyaniline, 4-methoxyaniline, 4-chloroaniline, p-toluidine, 4-nitroaniline, 3-bromoaniline, 3-bromo-4-aminotoluene, p-aminobenzoic acid, 2, 4-diaminotoluene, 2,5-dichloroaniline, 1,4-phenylene diamine, 4,4'-methylene dianiline, 1,3,5-triaminobenzene, and mixtures thereof, into reactive contact with nitrobenzene; and
(b) obtaining a 4-aminodiphenylamine intermediate product by reacting the aniline or aniline derivative and nitrobenzene in a confined zone at a suitable time, pressure and temperature, in the presence of a mixture comprising an organic base, and an inorganic salt or a metal organic salt, or mixtures thereof, selected from the group consisting of fluoride, chloride, bromide, sulfate, hydrogen sulfate, nitrate, phosphate, dihydrogen phosphate, formate, acetate, oxalate, malonate, citrate, tartrate, maleate, chlorate, perchlorate, chromate, rhenate and carbonate salts of cesium, rubidium, potassium and sodium, and mixtures thereof, and an oxidant, wherein the organic base is selected from the group consisting of tetramethylammonium hydroxide, tetrabutylammonium hydroxide, methyltributylammonium hydroxide, benzyltrimethylammonium hydroxide, tricaprylmethylammonium hydroxide, cetyltrimethylammonium hydroxide, and choline hydroxide; and
(c) reducing the 4-aminodiphenylamine intermediate product of step (b) to produce 4-aminodiphenylamine or substituted derivatives thereof.

### DETAILED DESCRIPTION OF THE INVENTION

The present invention is directed to a method, as described above, for making intermediates of 4-ADPA that has superior yield and selectivity for those intermediates. Such intermediates comprise 4-nitroso- and/or 4-nitrodiphenylamlnes (*p*-NDPA and 4-NDPA, respectively) and salts thereof. The intermediates may then be hydrogenated to produce 4-aminodiphenylamine.

The method of the present invention starts with an organic base and an inorganic salt or a metal organic salt as in the above embodiment.

Organic bases known or believed to be particularly effective include quaternary ammonium hydroxides selected from the group consisting of tetramethylammonium hydroxide, tetrabutylammonium hydroxide, methyltributylammonium hydroxide, benzyltrimethylammonium hydroxide (Triton B), tricaprylmethylammonium hydroxide, cetyltrimethylammonium hydroxide and choline hydroxide.

While aniline most effectively couples with nitrobenzene, certain aniline derivatives comprising amides such as formanilide, phenylurea and carbanilide as well as the thiocarbanilide can be substituted to produce 4-ADPA intermediates.

Although the reactants of the method of the invention are referred to as "aniline" and "nitrobenzene", and when it is 4-ADPA that is being manufactured the reactants are in fact aniline and nitrobenzene, it is understood that the reactants may also comprise substituted aniline selected from 2-methoxyaniline, 4-methoxyaniline, 4-chloroaniline, p-toluidine, 4-nitroaniline, 3-bromoaniline, 3-bromo-4-aminotoluene, p-aminobenzoic acid, 2,4-diaminotoluene, 2,5-dichloroaniline, 1,4-phenylene diamine, 4,4'-methylene dianiline, 1,3,5-triaminobenzene, and mixtures thereof.

The method of the invention includes the step wherein the 4-ADPA intermediates or substituted derivatives thereof from step (b) are subjected to a hydrogenation reaction involving the use of a hydrogenation catalyst. Details concerning choice of catalyst and other aspects of the hydrogenation reaction may be found in U.S. Patent No. 6,140,538.

Other means of reduction, that do not involve the direct use of hydrogen and are known to one skilled In the art, can also be used to reduce the 4-ADPA intermediates or substituted derivatives thereof to 4-ADPA or substituted derivatives thereof.

The present invention further relates to a process for preparing alkylated derivatives of 4-aminodiphenylamines, in particular for preparing alkyl derivatives of 4-ADPA itself, which are useful for the protection of rubber products, in which process an optionally substituted aniline and nitrobenzene are coupled and subsequently reduced according to the invention process, after which the 4-aminodiphenylamine so obtained is reductively alkylated to an alkylated derivative of the 4-aminodiphenylamine according to methods known to the person skilled in this technical field. Typically, the 4-ADPA and a suitable ketone, or aldehyde, are reacted in the presence of hydrogen and platinum-on-carbon as catalyst. Suitable ketones include methylisobutyl ketone, acetone, methylisoamyl ketone, and 2-octanone. See for example U.S. 4,463,191, and Banerjee et al. J. Chem. Soc. Chem. Comm. 18, 1275-1276 (1988). Suitable catalysts can be the same as, but not limited to, those described above for obtaining the 4-ADPA.

In a preferred embodiment of the invention, the reduction is conducted in the presence of water, e.g. water is added to the reaction mixture. The use of water is particularly advantageous when the suitable base, used during the reaction of the aniline or substituted aniline derivative and the nitrobenzene, is water-soluble. When the base is water- soluble, the amount of water added is preferably at least the amount needed to extract the base from the organic phase. Similarly, the addition of water is also preferred for reductive alkylation, if it is carried out in the presence of the suitable base, which is water-soluble.

The molar ratio of aniline to nitrobenzene in the process according to the present invention is not particularly important, as the process will be effective with an excess of either.

Inorganic salts and metal organic salts that may be used in conjunction with the organic base in the process of the invention have a cation that would be a suitable cation of a strong inorganic base. These inorganic salts and metal organic salts are selected from the group consisting of the fluoride, chloride, bromide, sulfate, hydrogen sulfate, nitrate, phosphate, dihydrogen phosphate, formate, acetate, oxalate, malonate, citrate, tartrate, maleate, chlorate, perchlorate, chromate, rhenate and carbonate salts of cesium, rubidium, potassium and sodium. In the method of the invention, the inorganic salt or metal organic salt may be used in molar ratio to nitrobenzene from 0.05:1 to 6.5:1.

Inorganic salts and metal organic salts known or believed to be particularly effective In the method of the present invention are those that afford acceptable solubility for the inorganic salt or metal organic sail-organic base combination in the reaction medium, including the fluoride, chloride, bromide, sulfate, hydrogen sulfate, nitrate, phosphate, formate, acetate and carbonate salts of cesium, rubidium, potassium and sodium and mixtures thereof. It is preferred that mole ratio of organic base used with an inorganic salt or metal organic salt to nitrobenzene is greater than or equal to 1:1. It is also preferred that mole ratio of inorganic salt or metal organic salt to organic base is greater than or equal to 1:1. A particularly preferred mole ratio of organic base to nitrobenzene is 1.1:1 to 6:1.

It may be desirable to use a combination of an inorganic salt with a metal organic salt, two or more inorganic salts and/or two or more metal organic salts in case one of the salts that is otherwise effective for use in the process of the invention has a corrosive effect on the equipment used with the process. The combination might also provide better results than could be obtained with one salt.

The use of inorganic salts and metal organic salts with the organic base is also believed to reduce undesirable base decomposition.

In the process according to the invention, it should be noted that an organic base with an inorganic salt or a metal organic salt will give some In situ formation of the equivalent inorganic base and a phase transfer catalyst, wherein the anion in formula I for the so formed phase transfer catalyst is the anion from the salt. For example, tetramethylammonium hydroxide plus potassium bromide will give some KOH plus tetramethylammonium bromide. This corresponds to the direct use of an inorganic base with any phase transfer catalyst that can be formed in situ, such as tetramethylammonium bromide, in lieu of tetramethylammonium hydroxide and a bromide salt as separate ingredients.

A particularly preferred combination of organic base and inorganic salt is tetraalkylammonium hydroxide and a salt in which the anion is a halide, such as potassium halide. A preferred halide anion is chloride. The above reactions would be carried out in aqueous solution with a continuous distillation of aniline-water azeotrope.

The reactive contact of the process of the invention is carried out in the presence of an oxidant. The oxidant may be free oxygen, or comprise an oxidizing agent such as a peroxide, particularly hydrogen peroxide. Nitrobenzene may also function as an oxidizing agent.

In die process of the invention, the oxidant may advantageously need to be present only for part of the time during which the aniline and nitrobenzene react. Such partial oxidative conditions are particularly effective for improving selectivity. One of these instances is when an inorganic salt with a fluoride anion is employed in the reaction mixture under partial oxidative conditions. It is believed that better results, conversion and selectivity, would also be obtained under partial oxidative conditions when the salt anion is sulfate, carbonate, or nitrate and other anions that give relatively low selectivity.

The reactive contact may be carried out at a temperature of from 20°C to 150°C. Other conditions for the reactive contact include pressures In the range of from 20 mbar to 20 barg. Reaction time is typically less than 3.5 hours, It is advantageous to agitate the reaction mixture during the entire reaction.

The reaction of step (b) of the present method may be carried out in the presence of not greater than 10:1 moles water to moles nitrobenzene. The amount of water does not include the water that hydrates with the reactants and/or with compounds formed in the process. When the mixture comprising an organic base and Inorganic Salt or metal organic salt, is in aqueous solution, the reaction may be carried out with a continuous distillation of aniline-water azeotrope.

The aqueous phase may be reused to form a new reaction mixture. Fresh organic base and inorganic salt or metal organic salt are added to replace losses by decomposition, by-product formation and solubility in the separated organic phase. Excess aniline recovered by distillation from the reaction product mixture may be combined with make-up fresh aniline for recycle to form a new reaction mixture. Recovery of excess nitrobenzene is preferably carried out prior to hydrogenation of the 4-ADPA intermediate by a separation step and the recovered nitrobenzene may be combined with make-up fresh nitrobenzene for use in the process, or hydrogenated to aniline.

The method of the present invention for the preparation of 4. aminodiphenylamines intermediates may be conducted as a batch process or may be performed continuously using means and equipment well known to the skilled person.

The reactive contact in step (a) of the method of the invention may occur in a suitable solvent system. A suitable solvent system comprises a polar aprotic solvent. The polar aprotic solvent may be selected form the group consisting of, but not limited to, dimethyl sulfoxide, benzyl ether, 1-Methyl-2-pyrrolidinone and N,N-dimethylformamide.

The invention is illustrated by the following examples. Examples 1-11 and 16 are not according to the invention.

Experimental conditions are detailed within individual examples. In examples 1-10 the charging of reactors was performed in open air resulting in some free oxygen being present during the reactions, even when the reactor was stoppered, except for experiments, where indicated, run for comparative purposes. No attempt was made to remove water from the reaction mixtures in examples 1-10.

In examples 11-16 a flow of air was supplied to the reactor headspace during all or part of charging reactants, heat-up to reaction temperature, nitrobenzene feed and hold, resulting in free oxygen being present during the reaction, except where indicated. Water was removed from the reaction mixture by azeotropic distillation with aniline. However, the reaction can also be effective without the azeotropic removal of water with aniline.

### ANALYTICAL

Yields of individual components were determined by external standard HPLC. Approximately 0.6 grams of material to be analyzed is accurately weighed into a 50-mL volumetric flask and diluted with a buffer solution containing 39% v/v water, 36% v/v acetonitrile, 24% v/v methanol and 1% v/v pH 7 buffer. The solution is injected through a 10 µL loop onto a reversed phase Zorbax ODS HPLC column (250 x 4.6 mm) using a binary gradient pumping system and the following elution gradient at a constant flow rate of 1.5 mL/minute:

| Time, minutes | %A | %B |
|---|---|---|
| 0 | 100 | 0 |
| 25 | 25 | 75 |
| 35 | 0 | 100 |
| 37.5 | 0 | 100 |
| 38 | 100 | 0 |
| 40 | 100 | 0 |

Eluent A is 75% v/v water, 15% v/v acetonitrile and 10% v/v methanol. Eluent B is 60% v/v acetonitrile and 40% v/v methanol. Detection is UV at 254 nm.

Conversion for examples 1-10 is calculated by sum addition of known components plus any unknown peaks (assigned an arbitrary mole weight value of 216, aniline + nitrobenzene) as analyzed. In some instances, sum conversion is greater than 100% due to the formation of derivatives from aniline only.

Conversion for examples 11-16 is calculated based on the amount of unreacted nitrobenzene remaining in the final coupling reaction mass. Conversion is assumed to be 100% if no nitrobenzene is detected.

Selectivity is defined by the formula: (p-NDPA Yield + 4-NDPA Yield)/(total yield). 4-NDPA is 4-nitrodiphenylamine and p-NDPA is 4-nitrosodiphenylamine. Total yield is the sum of the yield of all known and unknown peaks (assigned an arbitrary mole weight value of 216, aniline + nitrobenzene).

In the tables: "An Recr" refers to compounds from which aniline may be easily recovered and is a sum total of trans-azobenzene and azoxybenzene; "Others" are aniline and nitrobenzene coupling by-products e.g. phenazine, N-oxy-phenazine, 2-NDPA, 4-phenazo-diphenylamine and any unknowns.

### EXPERIMENTAL

Experimental conditions are detailed within individual examples.

### EXAMPLE 1 (comparative)

Example 1 illustrates that 4-ADPA intermediates may be formed from aniline and nitrobenzene in the presence of an inorganic base (potassium hydroxide) and phase transfer catalyst (tetramethylammonium chloride, TMACI) in a solvent-free system under relatively mild conditions. Yield of desired products is dependent on the amount of phase transfer catalyst added.

Aniline (99%, 22.58 grams, 240 mmoles), nitrobenzene (99%, 4.97 grams, 40 mmoles), potassium hydroxide (86% ground powder, 7.83 grams, 120 mmoles) and tetramethylammonium chloride were charged to a 50-mL round bottom flask equipped with magnetic stirrer in the amount indicated in Table 1 below. The reaction was allowed to proceed for 1 hour at 60°C in a stoppered flask. Contents were then sampled and analyzed by HPLC.

**Table 1**

| Yield, % | | | | | |
|---|---|---|---|---|---|
| | Conversion | *p*-NDPA | 4-NDPA | An Recr | Other |
| No TMACI added, KOH Only | 26.3% | 0.8 | 4.8 | 5.5 | 15.2 |
| 1.81 grams TMACI, 16 mmoles (0.4 vs NB) | 59.2% | 10.9 | 26.9 | 18.4 | 3.0 |
| 3.62 grams TMACI, 32 mmoles (0.8 vs NB) | 90.1 % | 22.4 | 36.1 | 28.6 | 3.0 |
| 5.42 grams TMACI, 48 mmoles (1.2 vs NB) | 98.2% | 27.0 | 37.8 | 30.3 | 3.0 |
| 7.23 grams TMACI, 64 mmoles (1.6 vs NB) | 94.4% | 26.5 | 36.2 | 28.9 | 2.9 |
| 9.04 grams TMACI, 80 mmoles (2.0 vs NB) | 98.9% | 26.2 | 36.7 | 31.8 | 4.2 |

Similar results were obtained when running the reaction under slightly varying conditions (equimolar An/NB, higher reaction temperature, longer cycle time, water addition, etc.) as given below in Table 2.

Aniline (99%, 2.33 grams, 24.8 mmoles), nitrobenzene (99%, 3.08 grams, 24.8 mmoles), potassium hydroxide (86% ground powder, 9.77 grams, 150 mmoles), tetramethylammonium chloride (97%, see Table 2) and water (Table 2) were charged to a 50-mL round bottom flask equipped with magnetic stirrer. The water amount was 20% by weight of total reactor charge assuming 14% w/w H₂O from KOH. The reaction was allowed to proceed for 2 hours at 80°C in an open flask. Contents were then sampled and analyzed by HPLC.

**Table 2**

| Yield, % | | | | | |
|---|---|---|---|---|---|
| | Conversion | *p*-NDPA | 4-NDPA | An Recr | Other |
| No TMACl, KOH Only & 2.15 g H₂O | 2.3% | 0.0 | 0.4 | 0.4 | 1.5 |
| 0.17 g TMACl, 1.5 mmoles (.06 vs NB) & 2.19 g H₂O | 8.1% | 0.3 | 5.9 | 0.7 | 1.2 |
| 0.34 g TMACl, 3.0 mmoles (.12 vs NB) & 223 g H₂O | 14.7% | 0.7 | 12.7 | 0.3 | 1.0 |
| 0.69 g TMACl, 6.1 mmoles (.25 vs NB) & 2.32 g H₂O | 34.4% | 1.7 | 27.7 | 2.9 | 2.1 |
| 1.03 g TMACl, 9.1 mmoles (.37 vs NB) & 2.41 g H₂O | 47.5% | 1.6 | 39.5 | 4.2 | 2.2 |
| 1.37 g TMACl, 12.1 mmoles (.49 vs NB) & 2.49 g H₂O | 57.8% | 2.6 | 46.7 | 5.2 | 3.3 |
| 2.06 g TMACl, 18.2 mmoles (.74 vs NB) & 2.67 g H₂O | 89.6% | 7.6 | 61.3 | 17.7 | 3.0 |
| 2.74 g TMACl, 24.3 mmoles (.98 vs NB) & 2.84 g H₂O | 92.2% | 11.9 | 64.9 | 13.4 | 2.0 |

The yield, of 4-ADPA intermediates was increased from < 1% when no tetramethylammonium chloride was used to almost 77% when a near equimolar amount of phase transfer catalyst vs. nitrobenzene was added.

In both instances, more p-NDPA relative to 4-NDPA was produced as the tetramethylammonium chloride charge was increased. Also, more p-NDPA was formed in the presence of excess aniline (see Example 7).

### EXAMPLE 2 (comparative)

Example 2 demonstrates that any of several phase transfer catalysts may be used with KOH to produce p-NDPA and 4-NDPA from aniline and nitrobenzene. Results are arranged in order of descending yield.

Charge to 50-mL round bottom flask equipped with magnetic stirrer: aniline (99%, 22.58 grams, 240 mmoles), nitrobenzene (99%, 4.97 grams, 40 mmoles), potassium hydroxide (86% ground powder, 7.83 grams, 120 mmoles) and the indicated phase transfer catalyst given in Table 3 below where the amount of phase transfer catalyst is equal to the limiting reagent charge. (NOTE: Some experiments run on 20 or 30 mmole scale as denoted.) Reaction was allowed to proceed for 1 hour at 60°C in a stoppered flask. Contents were then sampled and analyzed by HPLC.

Results in Table 3 above illustrate that the addition of a phase transfer catalyst improves the yield of desired products in all cases. Tetramethylammonium chloride, fluoride, hydroxide, carbonate, formate and acetate; tetrabutylammonium hydrogensulfate and sulfate; methyltributylammonium chloride; and benzyltrimethylammonium hydroxide (Triton B) are most effective as phase transfer catalysts in combination with an inorganic base. Others such as tricaprylmethylammonium chloride (Aliquat 336), tetrabutylammonium chloride, tetramethylammonium nitrate, and choline hydroxide are moderately efficient. Bromide and iodide salts and the zwitterion betaine are not as suitable. Periodic trends are observed for the tetramethylammonium salts as yield, conversion and selectivity are all decreased when going down in the series from fluoride to iodide.

**Table 3**

| Yield, % | | | | | |
|---|---|---|---|---|---|
| | Conversion | *p*-NDPA | 4-NDPA | An Recr | Other |
| Tetrabutylammonium sulfate, 75% aq., 15.49 gms^ | 99.2% | 56.1 | 21.2 | 20.1 | 1.8 |
| Tetrabutylammonium hydrogensulfate, 97%, 10.50 gms* | 96.6% | 47.9 | 25.0 | 21.2 | 2.4 |
| Tetramethylammonium carbonate, 60% aq., 6.94 gms^ | 97.6% | 46.6 | 24.6 | 23.7 | 2.8 |
| Tetramethylammonium fluoride · 4 H₂O, 98%, 6.74 gms | 103.6% | 42.4 | 27.2 | 27.4 | 6.6 |
| Tetramethylammonium acetate, 95%, 5.61 gms | 104.3% | 25.9 | 43.1 | 35.0 | 0.4 |
| Tetramethylammonium hydroxide · 5 H₂O, 97%, 7.47 gms | 105.0% | 38.4 | 29.6 | 30.4 | 6.5 |
| Tetramethylammonium chloride, 97%, 4.52 gms | 98.8% | 24.3 | 37.1 | 30.8 | 6.6 |
| Methyltributylammonium chloride, 75% aq., 12.58 gms | 81.7% | 23.5 | 30.6 | 22.2 | 5.4 |
| Tetramethylammonium formate, 50% aq., 9.53 gms | 74.9% | 29.0 | 23.8 | 21.0 | 1.1 |
| Benzyltrimethylammonium hydroxide, 40% aq., 16.73 gms | 52.5% | 39.6 | 6.2 | 5.5 | 1.2 |
| Tricaprylmethylammonium chloride, 99+%, 16.17 gms | 67.0% | 19.1 | 21.3 | 19.6 | 7.0 |
| Tetramethylammonium nitrate, 96%, 5.67 gms | 61.3% | 12.0 | 27.4 | 19.6 | 2.4 |
| Choline hydroxide, 50% aq., 9.69 gms | 59.0% | 26.2 | 6.7 | 19.6 | 6.6 |
| Tetrabutylammonium chloride · H2O, 98%, 8.51 gms* | 42.6% | 8.2 | 23.8 | 9.3 | 1.2 |
| Betaine, 98%, 4.78 gms | 55.0% | 13.0 | 17.2 | 19.6 | 5.2 |
| Cetyltrimethylammonium bromide, 95%, 11.51 gms* | 36.2% | 7.0 | 19.3 | 8.7 | 1.1 |
| Tetramethylammonium bromide, 98%, 6.29 gms | 36.5% | 11.3 | 12.2 | 6.7 | 6.3 |
| Tetrabutylammonium bromide, 99%, 13.03 gms | 34.1% | 8.3 | 14.2 | 5.8 | 5.7 |
| Polyethylene glycol (MW ≈ 200), 8.00 gms | 33.4% | 11.9 | 0.6 | 17.2 | 3.7 |
| Tetramethylammonium iodide, 99%, 8.12 gms | 27.8% | 2.4 | 8.0 | 5.8 | 11.6 |
| Tetrabutylphosphonium bromide, 98%, 13.58 gms | 25.6% | 1.6 | 5.1 | 9.4 | 10.0 |
| KOH Only, No phase transfer catalyst added | 19.6% | 1.2 | 4.0 | 3.3 | 11.0 |

| | | | | | |
|---|---|---|---|---|---|
| *30 mmole scale (16.93 gms aniline, 3.73 gms nitrobenzene, 5.87 gms KOH & PTC as listed) ^20 mmoles (TMA)₂CO₃ & (TBA)₂SO₄ (0.5 to 1 vs NB, same no. of equivalents) | | | | | |

### EXAMPLE 3

### Example 3 (comparative) shows that nitrobenzene may be coupled with a variety of aniline derivatives to produce 4-ADPA intermediates.

A stoichiometric amount of substrate as listed in Table 4 below; nitrobenzene (99%, 3.08 grams, 24.8 mmoles), potassium hydroxide (86% ground powder, 9.77 grams, 150 mmoles), tetramethylammonium chloride (97%, 2.74 grams, 24.3 mmoles), and water (2.84 grams) were charged to a 50-mL round bottom flask equipped with a magnetic stirrer. The reaction was allowed to proceed for 2 hours at 80°C in an open flask. Contents were then sampled and analyzed by HPLC.

**Table 4**

| Yield, % | | | | | |
|---|---|---|---|---|---|
| | Conversion | p-NDPA | 4-NDPA | An Recr | Other |
| Aniline, 99%, 2.33 grams, 24.8 mmoles | 95.4% | 6.0 | 68.3 | 19.9 | 1.2 |
| Formanilide, 99%, 3.03 grams, 24.8 mmoles | 84.5% | 19.3 | 47.3 | 16.3 | 1.5 |
| Phenylurea, 97%, 3.40 grams, 24.2 mmoles | 96.2% | 19.2 | 38, 8 | 13.5 | 24.8 |
| Carbanilide, 98%, 2.65 grams, 12.2 mmoles | 48.1% | 1.3 | 37.1 | 9.3 | 0.4 |
| Thiocarbanilide, 98%, 2.85 grams, 12.2 mmoles | 58.6% | 5-4 | 31.6 | 18.6 | 3.0 |
| Acetanilide, 97%, 3.38 grams, 24.3 mmoles | 8.5% | 0.3 | 2.7 | 3.6 | 1.9 |
| Benzamide, 99%, 3.03 grams, 24.8 mmoles | 49.4% | 0.0 | 1,0 | 15.1 | 33.3 |
| N-Methyl-Benzamide, 99+%, 3.38 grams, 25.0 mmoles | 8.2% | 0.0 | 0.0 | 0.0 | 8.2 |
| Benzanilide, 98%, 2.47 grams, 12.3 mmoles | 0.1% | 0.0 | 0.1 | 0.0 | 0.0 |

While aniline most effectively couples with nitrobenzene in a KOH-TMACl system, amides such as formanilide, phenylurea and carbanilide as well as the thiocarbanilide can be substituted to produce 4-ADPA intermediates.

### EXAMPLE 4 (comparative)

Example 4 illustrates the reaction of aniline and nitrobenzene using various bases in combination with tetramethylammonium chloride to produce 4-ADPA intermediates.

Aniline (99%, 22.58 grams, 240 mmoles), nitrobenzene (99%, 4.97 grams, 40 mmoles), an appropriate amount of base as given in Table 5 below and tetramethylammonium chloride (97%, 4.52 grams, 40 mmoles) was charged to a 50-mL round bottom flask equipped with a magnetic stirrer. The reaction was allowed to proceed for 1 hour at 60°C in a stoppered flask. Contents were then sampled and analyzed by HPLC.

**Table 5**

| Yields, % | | | | | |
|---|---|---|---|---|---|
| | Conversion | *p*-NDPA | 4-NDPA | An Recr | Other |
| KOH, 86%, 7.83 grams, 120 mmoles (3:1 vs NB) | 97.1% | 25.2 | 35.5 | 30.6 | 5.8 |
| KOH, 86%, 13.05 grams, 200 mmoles (5:1 vs NB) | 100.5% | 21.5 | 36.0 | 32.0 | 11.0 |
| NaOH, 98%, 4,90 grams, 120 mmoles (3:1 vs NB) | 21.3% | 4.7 | 12.6 | 3.4 | 0.6 |
| NaOH, 98%, 8.16 grams, 200 mmoles (5:1 vs NB) | 50.4% | 11.5 | 24.5 | 14.2 | 0.2 |
| CsOH·H₂O, 95%, 15.91 grams, 90 mmoles (3:1 vs NB)⁺ | 98.8% | 20.5 | 43.2 | 34.5 | 0.6 |
| t-BuOK, 95%, 11.84 grams, 100 mmoles (2½:1 vs MB) | 107.1% | 15.2 | 33.4 | 25.0 | 33.5 |
| TMAH-5H₂O, 22.42 grams, 120 mmoles (3:1 vs NB)^ | 51.5% | 38.2 | 7.0 | 5.9 | 0.4 |

| | | | | | |
|---|---|---|---|---|---|
| *30 mmole scale (16.93 gms aniline, 3.73 gms nitrobenzene, 3.39 gms TMACl & base as indicated) Tetramethylammonium hydroxide only. No TMACl added. | | | | | |

Both lithium and calcium hydroxide were screened with no reaction observed for either of these two bases.

Potassium hydroxide is the preferred base but sodium hydroxide, cesium hydroxide, potassium t-butoxide and tetramethylammonium hydroxide are also suitable bases any of which may used in combination with tetramethylammonium chloride to obtain acceptable rates of conversion.

### EXAMPLE 5 (comparative)

Example 5 demonstrates the effect of increasing potassium hydroxide charge on aniline-nitrobenzene coupling products under otherwise constant reaction conditions with tetramethylammonium chloride as a phase transfer catalyst.

Aniline (99%, 22.58 grams, 240 mmoles), nitrobenzene (99%, 4.97 grams, 40 mmoles), potassium hydroxide in the amount given in Table 6 below and tetramethylammonium chloride (97%, 4.52 grams, 40 mmoles) was charged to a 50-mL round bottom flask equipped with magnetic stirrer. The reaction was allowed to proceed for 1 hour at 60°C in a stoppered flask. Contents were then sampled and analyzed by HPLC.

**Table 6**

| Yield, % | | | | | |
|---|---|---|---|---|---|
| | Conversion | p-NDPA | 4-NDPA | An Recr | Other |
| No KOH, TMACl Only | 0.0% | 0.0 | 0.0 | 0.0 | 0.0 |
| 1.30 grams KOH, 20 mmoles (0.5:1 vs NB) | 54.9% | 18.8 | 19.6 | 15.7 | 0.7 |
| 2.61 grams KOH, 40 mmoles (1:1 vs NB) | 69.2% | 21.3 | 26.8 | 20.8 | 0.3 |
| 5.22 grams KOH, 80 mmoles (2:1 vs NB) | 91.8% | 26.0 | 33.5 | 29.1 | 3.2 |
| 7.83 grams KOH, 120 mmoles (3:1 vs NB) | 97.1% | 25.2 | 35.5 | 30.6 | 5.8 |
| 10.44 grams KOH, 160 mmoles (4:1 vs NB) | 99.1% | 23.6 | 36.0 | 32.0 | 7.5 |
| 13.05 grams KOH, 200 mmoles (5:1 vs NB) | 100.5% | 21.5 | 36.0 | 32.0 | 11.1 |
| 15.66 grams KOH, 240 mmoles (6:1 vs NB) | 101.7% | 18.4 | 33.6 | 32.7 | 17.0 |

Higher excesses of base result in poorer reaction selectivity and more by-product formation. The same trend is observed when running the reaction under comparatively milder reaction conditions as described in Table 7 below. Similarly, conversion is a function of the amount of base used.

Aniline (99%, 32.60 grams, 346.5 mmoles), nitrobenzene (99%, 6.16 grams, 49.5 mmoles), potassium hydroxide in the amount given in Table 7 below (86% ground powder, 16.31 grams, 250 mmoles) and tetramethylammonium chloride (97%, 5.48 grams, 48.5 mmoles) were charge to a 100-mL round bottom flask equipped with a Teilon paddle stirrer. The reaction was allowed to proceed for 1 hour with no application of external heat (some exotherm generated by dissolution of KOH in reaction water) in a stoppered flask. Contents were then sampled and analyzed by HPLC.

**Table 7**

| Yield, % | | | | | |
|---|---|---|---|---|---|
| | Conversion | *p*-NDPA | 4-NDPA | An Recr | Other |
| 9.77 grams KOH, 150 mmoles (3:1 vs NB) | 10.5% | 1.3 | 8.6 | 0.0 | 0.6 |
| 13.05 grams KOH, 200 mmoles (4:1 vs NB) | 64.6% | 14.9 | 26.2 | 15.4 | 8.1 |
| 16.31 grams KOH, 250 mmoles (5:1 vs NB) | 92.2% | 21.8 | 33.0 | 27.0 | 10.4 |
| 19.57 grams KOH, 300 mmoles (6:1 vs NB) | 100.5% | 21.7 | 33.6 | 31.8 | 13.5 |
| 22.84 grams KOH, 350 mmoles (7:1 vs NB) | 104.4% | 21.3 | 33.6 | 33.5 | 16.0 |

### EXAMPLE 6 (comparative)

Example 6 indicates the effect that the introduction of an oxidant has on the conversion of aniline and nitrobenzene to p-NDPA, 4-NDPA and by-products when utilizing a potassium hydroxide / tetramethylammonium chloride base-PTC system.

Aniline (99%, 2.33 grams, 24.8 mmoles), nitrobenzene (99%, 3.08 grams, 24.8 mmoles), potassium hydroxide (86% ground powder, 9.77 grams, 150 mmoles), tetramethylammonium chloride (97%, 0.69 grams, 6.1 mmoles) and water (2.32 grams) were charged to a 50-mL round bottom flask equipped with a magnetic stirrer. The reaction was allowed to proceed for 2 hours at 80°C under atmospheric conditions described below. Contents were then sampled and analyzed by HPLC.

The definition of a closed system is a stoppered flask. An open system is left unstoppered and open to the atmosphere. For gas sweep experiments, a three-necked flask is substituted for a single-necked flask, the system equipped with both a gas inlet and outlet line, and the appropriate gas swept across the reaction mass at a low flow rate.

**Table 8**

| Yield, % | | | | | | |
|---|---|---|---|---|---|---|
| | Conversion | Selectivity | *p*-NDPA | 4-NDPA | An Recr | Other |
| Closed System | 45.1 % | 61.3% | 1.4 | 26.3 | 15.9 | 1.5 |
| Open System | 34.4% | 85.6% | 1.7 | 27.7 | 2.9 | 2.1 |
| Gas Sweep, Nitrogen | 94.8% | 58.2% | 2.4 | 52.8 | 38.3 | 1.3 |
| Gas Sweep, Air | 60.6% | 86.8% | 2.8 | 49.8 | 3.3 | 4.7 |

In cases where the reaction is left open to excess air, selectivity is markedly improved, as opposed to experiments where the amount of oxidant is limited. Formation of azobenzene is greatly increased in the latter instance.

Improvement in reaction selectivity is reinforced by experiments in Table 9, which demonstrate the effect of hydrogen peroxide addition in the reaction mixture.

Aniline (99%, 22.58 grams, 240 mmoles), nitrobenzene (99%, 4.97 grams, 40 mmoles), hydrogen peroxide (50% aqueous, amount indicated in Table 9 below), water (sum total from additional water and peroxide kept constant at 2.16 grams), potassium hydroxide (86% ground powder, 7.83 grams, 120 mmoles) and tetramethylammonium chloride (97%, 4.52 grams, 40 mmoles) was charged to a 50-mL round bottom flask equipped with a magnetic stirrer. Peroxide was charged to the reaction mixture before adding KOH & TMACI with the flask quickly stoppered and then the reaction was allowed to proceed for 1 hour at 60°C. Contents were then sampled and analyzed by HPLC.

**Table 9**

| Yield, % | | | | | | |
|---|---|---|---|---|---|---|
| | Conversion | Selectivity | *p*-NDPA | 4-NDPA | An Recr | Other |
| No H₂O₂ & 2-16 g water | 96.6% | 67.9% | 27.9 | 37.7 | 30.6 | 0.5 |
| 0.27 g H₂O₂, 4 mmol, (0.1 vs NB) & 2.02 g water | 90.3% | 73.8% | 27.3 | 39.4 | 23.0 | 0.7 |
| 0.54 g H₂O₂, 8 mmol, (0.2 vs NB) & 1.89 g water | 86.6% | 77.7% | 27.3 | 40.0 | 18.3 | 1.0 |
| 1.09 g H₂O₂, 16 mmol, (0.4 vs NB) &1.62 g water | 86.4% | 77.3% | 25.5 | 41.3 | 18.3 | 1.3 |
| 1.63 g H₂O₂, 24 mmol, (0.6 vs NB) & 1.34 g water | 86.4% | 78.4% | 26.9 | 40.9 | 17.6 | 1.1 |
| 2.18 g H₂O₂, 32 mmol, (0.8 vs NB) & 1.07 g water | 79.8% | 80.3% | 25.6 | 38.4 | 14.3 | 1.4 |
| 272 g H₂O₂, 40 mmol, (1.0 vs NB) & 0.80 g water | 80.8% | 82.0% | 25.9 | 40.4 | 13.0 | 1.6 |

The same trend noted for opening the reaction contents to air is also seen for peroxide, namely exposure to an oxidant improves selectivity. This observation is reinforced by experimental trials where excess nitrobenzene is used to act as an oxidant. (see Example 7).

### EXAMPLE 7 (comparative)

Example 7 shows how the ratio of 4-ADPA intermediates can be controlled by adjusting the amount of aniline charged into the reaction.

Aniline (99%, amount given in Table 10), nitrobenzene (99%, 4.97 grams, 40 mmoles), potassium hydroxide (86% ground powder, 7.83 grams, 120 mmoles) and tetramethylammonium chloride (97%, 4.52 grams, 40 mmoles) was charged to 50-mL round bottom flask equipped with magnetic stirrer. The reaction was allowed to proceed for 1 hour at 60°C in a stoppered flask. Contents were then sampled and analyzed by HPLC.

**Table 10**

| Yield, % | | | | | | |
|---|---|---|---|---|---|---|
| | Conv | Ratio | *p*-NDPA | 4-NDPA | An Recr | Other |
| 35.28 grams Aniline, 375 mmoles, 15:1 vs NB* | 87.2% | 1.34 | 36.7 | 27.3 | 22.2 | 1.1 |
| 36.69 grams Aniline, 390 mmoles, 13:1 vs NB^ | 93.2% | 1.31 | 37.1 | 28.4 | 26.3 | 1.4 |
| 36.22 grams Aniline, 385 mmoles, 11:1 vs NB# | 94.7% | 1.14 | 35.2 | 30.9 | 26.5 | 2.2 |
| 33.87 grams Aniline, 360 mmoles, 9:1 vs NB | 95.4% | 0.96 | 32.0 | 33.2 | 27.5 | 2.6 |
| 26.34 grams Aniline, 280 mmoles, 7:1 vs NB | 96.8% | 0.75 | 27.1 | 36.0 | 30.3 | 3.5 |
| 18.81 grams Aniline, 200 mmoles, 5:1 vs NB | 95.9% | 0.60 | 23.1 | 38.8 | 31.1 | 2.8 |
| 11.29 grams Aniline, 120 mmoles, 3:1 vs NB | 92.3% | 0.37 | 15.7 | 42.4 | 30.9 | 3.4 |
| 3.76 grams Aniline, 40 mmoles, 1:1 vs NB | 80.1% | 0.14 | 6.1 | 43.8 | 24.7 | 5.5 |

| | | | | | | |
|---|---|---|---|---|---|---|
| *25 mmole scale (35.28 gms aniline, 3.11 gms nitrobenzene, 4.89 gms KOH & 2.82 gms TMACI) ^30 mmole scale (36.69 gms aniline, 3.73 gms nitrobenzene, 5.87 gms KOH & 3.39 gms TMACI) #35 mmole scale (36.22 gms aniline, 4.35 gms nitrobenzene, 6.85 gms KOH & 3.95 gms TMACI) | | | | | | |

As more aniline is charged to the reaction, more *p*-NDPA is formed relative to 4-NDPA. The same trend is noted under differing reaction conditions as outlined in Table 11 below.

Aniline (99%, amount given in Table 11), nitrobenzene (99%, 3.08 grams, 24.8 mmoles), potassium hydroxide (86% ground powder, 9.77 grams, 150 mmoles), tetramethylammonium chloride (97%, 0.69 grams, 6.1 mmoles) and water (Table 11, 20% w/w) were charged to a 50-mL round bottom flask equipped with a magnetic stirrer. The reaction was allowed to proceed for 2 hours at 80°C in an open flask. Contents were then sampled and analyzed by HPLC.

**Table 11**

| Yield, % | | | | | | |
|---|---|---|---|---|---|---|
| | Conv | Ratio | *p*-NDPA | 4-NOPA | An Recr | Other |
| 12.48 g An, 133 mmol, (5.4 vs NB) & 4.89 g H₂O | 18.6% | 0.52 | 5.6 | 10.9 | 1.0 | 1.1 |
| 8.57 g An, 91.1 mmol, (3.7 vs NB) & 3.90 g H₂O | 26.6% | 0.37 | 6.5 | 17.7 | 1.4 | 0.8 |
| 4.66 g An, 49.6 mmol, (2 vs NB) & 2.91 g H₂O | 28.9% | 0.12 | 2.6 | 20.9 | 3.2 | 2.1 |
| 2.33 g An, 24.8 mmol, (1 vs NB) & 2.32 g H₂O | 34.4% | 0.06 | 1.7 | 27.7 | 2.9 | 2.1 |
| 1.75 g An, 18.6 mmol, (.75 vs NB) & 2.17 g H₂O | 42.6% | 0.05 | 1.8 | 34.6 | 4.2 | 2.1 |
| 1.16 g An, 12.3 mmol, (.50 vs NB) & 2.02 g H₂O | 56.1% | 0.02 | 0.8 | 51.7 | 1.1 | 2.5 |
| 0.58 g An, 6.2 mmol, (.25 vs NB) & 1.88 g H₂O | 76.7% | 0.01 | 0.9 | 72.9 | 1.1 | 1.8 |

Yields of 4-ADPA intermediates (*p*-NDPA + 4-NDPA) remain relatively flat when aniline is used in excess (approx. 20%) but improve significantly (73.8% at 0.25 to 1 An/NB) when aniline becomes the limiting reagent as noted in Table 11. Also, selectivity is improved (96.1% at 0.25 to 1 An/NB) when nitrobenzene is used in excess despite less overall water. As shown in Example 9, less water typically decreases selectivity in an inorganic base system. Excess nitrobenzene here acts as an oxidant, improving selectivity as shown in Example 6 with air and peroxide.

### EXAMPLE 8 (comparative)

Example 8 illustrates that the reaction between aniline and nitrobenzene using potassium hydroxide as a base in conjunction with tetramethylammonium chloride can be conducted over a wide range of temperatures.

Aniline (99%, 2.33 grams, 24.8 mmoles), nitrobenzene (99%, 3.08 grams, 24.8 mmoles), potassium hydroxide (86% ground powder, 9.77 grams, 150 mmoles), tetramethylammonium chloride (97%, 0.69 grams, 6.1 mmoles) and water (2.32 grams, 20% w/w) were charged to a 50-mL round bottom flask equipped with magnetic stirrer. The reaction was allowed to proceed for 2 hours at the given temperature in an open flask. Contents were then sampled and analyzed by HPLC.

**Table 12**

| Yield, % | | | | | |
|---|---|---|---|---|---|
| | Conversion | *p*-NDPA | 4-NDPA | An Recr | Other |
| Reaction Temperature, 20°C | 9.3% | 0.1 | 8.3 | 0.0 | 1.0 |
| Reaction Temperature, 35°C | 21.6% | 0.5 | 19.5 | 0.2 | 1.4 |
| Reaction Temperature, 50°C | 25.2% | 0.8 | 22.3 | 0.1 | 1.9 |
| Reaction Temperature, 65°C | 26.0% | 0.6 | 22.8 | 0.4 | 2.2 |
| Reaction Temperature, 80°C | 34.4% | 1.7 | 27.7 | 2.9 | 2.1 |
| Reaction Temperature, 95°C | 39.3% | 2.3 | 27.8 | 7.5 | 1.7 |
| Reaction Temperature, 110°C | 53.8% | 3.5 | 33.4 | 12.8 | 4.0 |
| Reaction Temperature, 125°C | 72.7% | 9.1 | 34.0 | 17.3 | 12.4 |

Increasing reaction temperature results in improved yields and conversion but reaction selectivity is lost. The amount of *p*-NDPA, relative to 4-NDPA, increases with increasing temperature.

**Table 13**

| | Yield, % | Selectivity, % | *p*-NDPA/4-NDPA |
|---|---|---|---|
| Reaction Temperature, 20°C | 8.3 | 89.0 | 0.01 |
| Reaction Temperature, 35°C | 20.0 | 92.3 | 0.03 |
| Reaction Temperature, 50°C | 23.1 | 91.8 | 0.04 |
| Reaction Temperature, 65°C | 23.4 | 90.0 | 0.03 |
| Reaction Temperature, 80°C | 29.4 | 85.6 | 0.06 |
| Reaction Temperature, 95°C | 30.1 | 76.7 | 0.08 |
| Reaction Temperature, 110°C | 37.0 | 68.7 | 0.11 |
| Reaction Temperature. 125°C | 43.1 | 59.2 | 0.27 |

### EXAMPLE 9 (comparative)

Example 9 emphasizes the effect of water in the reaction of aniline and nitrobenzene with a KOH-TMACI base/phase transfer system to form 4-ADPA intermediates.

Aniline (99%, 22.58 grams, 240 moles), nitrobenzene (99%, 4.97 grams, 40 mmoles), potassium hydroxide (86% ground powder, 7.83 grams, 120 mmoles), tetramethylammonium chloride (97%, 4.52 grams, 40 mmoles), and water as listed in Tables 14 and 15 was charged to a 50-mL round bottom flask equipped with a magnetic stirrer. The reaction was allowed to proceed for 1 hour at 60°C in a stoppered flask. Contents were then sampled and analyzed by HPLC.

**Table 14**

| Yield, % | | | | | |
|---|---|---|---|---|---|
| | Conversion | *p*-NDPA | 4-NDPA | An Recr | Other |
| No Water added | 98.6% | 26.4 | 38.5 | 30.4 | 3.3 |
| 2.16 grams H₂O, 120 mmoles (3:1 vs NB) | 94.7% | 28.5 | 37.3 | 28.6 | 0.4 |
| 4.32 grams H₂O, 240 mmoles (6:1 vs NB) | 67.0% | 27.2 | 21.1 | 18.4 | 0.3 |
| 6.48 grams H₂O, 320 mmoles (9:1 vs NB) | 28.3% | 16.3 | 6.7 | 5.1 | 0.2 |
| 8.64 grams H₂O, 480 mmoles (12:1 vs NB) | 5.5% | 4.1 | 1.3 | 0.0 | 0.0 |

**Table 15**

| | Selectivity, % | *p*-NDPA / 4-NDPA |
|---|---|---|
| No Water added | 65.8 | 0.69 |
| 3:1 H₂O/NB (1 mole Water vs. KOH) | 69.4 | 0.77 |
| 6:1 H₂O/NB (2 moles Water vs. KOH) | 72.1 | 1.28 |
| 9:1 H₂O/NB (3 moles Water vs. KOH) | 81.3 | 2.44 |
| 12:1 H₂O/NB (4 moles Water vs. KOH) | 100.0 | 3.08 |

A general improvement in selectivity and higher levels of *p*-NDPA relative to 4-NDPA becomes evident as the amount of water is increased.

The effect of too much water may also be noted from Example 2 and Table 3 where the effectiveness of a 60% aqueous solution of tetramethylammonium carbonate as a phase transfer catalyst is shown. Previous unreported data obtained from a dilute 25% solution indicated practically no conversion.

### EXAMPLE 10 (comparative)

Example 10 shows that the reaction may be carried out in any of several solvents.

Aniline (99%, 11.29 grams, 120 mmoles), nitrobenzene (99%, 2.49 grams, 20 mmoles), potassium hydroxide (86% ground powder, 3.91 grams, 60 mmoles), tetramethylammonium chloride (97%, 2.26 grams, 20 mmoles) and 20-mL of the appropriate solvent as represented in Table 16 was charged to a 50-mL round bottom flask equipped with a magnetic stirrer. The reaction was allowed to proceed for 1 hour at 60°C in a stoppered flask. Contents were then sampled and analyzed by HPLC.

**Table 16**

| Yield, % | | | | | |
|---|---|---|---|---|---|
| | Conversion | *p*-NDPA | 4-NDPA | An Recr | Other |
| No solvent added | 97.1% | 25.2 | 35.5 | 30.6 | 5.8 |
| Dimethyl sulfoxide | 99.5% | 34.2 | 37.6 | 26.1 | 1.6 |
| Dimethyl sulfoxide, No phase transfer catalyst added | 36.5% | 10.9 | 15.8 | 6.4 | 3.4 |
| Benzyl ether | 93.7% | 30.6 | 32.1 | 28.1 | 3.0 |
| 1-Methyl-2-pyrrolidinone | 80.1% | 29.3 | 27.3 | 17.9 | 5.6 |
| N,N-Dimethylformamide | 74.0% | 27.2 | 27.2 | 19.1 | 0.6 |
| *p*-Xylene | 65.9% | 8.8 | 10.1 | 44.6 | 2.4 |
| Toluene | 63.3% | 3.0 | 3.7 | 51.1 | 5.5 |

Notable is a roughly two-thirds reduction in yield when the phase transfer catalyst is omitted (26.7% in DMSO without TMACI increasing to 71.8% with TMACI).

Selectivity remains relatively unchanged in polar solvents (~ 70%) but plunges significantly when non-polar hydrocarbons such as *p*-xylene or toluene are selected as azobenzene yields in each of these two solvents exceed 40%.

### EXAMPLE 11 (comparative)

Example 11 demonstrates the reaction of aniline and nitrobenzene in combination with an aqueous solution of potassium hydroxide and tetramethyalammonium chloride by continuous distillation of the aniline-water azeotrope.

111.8 grams aniline (99%, 1.19 moles), 31.2 grams aqueous potassium hydroxide solution (45%, 0.250 moles) and 50.0 grams aqueous tetramethylammonium chloride solution (55%, 0.25 moles) were charged to a 500-mL flask equipped with a Teflon paddle stirrer, thermocouple, nitrobenzene feed tube and needle valve. A vacuum was pulled on the mixture to 16 kPa (120 mm Hg), regulating pressure by bleeding air across the reactor. Heating was begun and nitrobenzene flow was started (24.6 grams, 99%, 0.20 moles) when the desired reaction temperature of 80°C was reached. The temperature was controlled by increasing the vacuum so as to complete the NB feed in approximately one hour at a final pressure of 8 kPa (60 mm Hg). The pressure was held for 45 minutes at 8 kPa (60 mm Hg) to insure completeness of reaction. The mixture was quenched with 40 mL of water. HPLC analysis: 32.1% aniline, 0% NB, 20.3% *p*-NDPA, 7.6% 4-NDPA, 0.50% *t*-azobenzene and 0.05% pheanzine. Yields based on 100% conversion of NB: 72.6% *p*-NDPA, 25.3%, 4-NDPA, 1.9% *t*-azobenzene, 0.2%, phenazine.

As shown in Table 17 below, running the identical reaction in the absence of air resulted in a 12% lower yield (97.9% vs. 85.5%) and a seven fold increase in the azobenzene level. A summary of other reactions in this series is also given in Table 17 below:

**Table 17**

| Yield, % | | | | | | |
|---|---|---|---|---|---|---|
| | Conversion | Selectivity | *p*-NDPA | 4-NDPA | *t*-Azo | Phenazine |

| BASELINE: | | | | | | |
|---|---|---|---|---|---|---|
| See conditions below* | 100.0% | 97.9% | 72.6 | 25.3 | 1.9 | 0.2 |

| ATMOSPHERE: | | | | | | |
|---|---|---|---|---|---|---|
| Baseline conditions (Vacuum, No Air) | 100.0% | 85.5% | 66.5 | 19.0 | 14.2 | 0.3 |

| ANILINE CHARGE: | | | | | | |
|---|---|---|---|---|---|---|
| 74.5 gms Aniline, 0.79 moles, 4:1 vs NB | 99.5% | 96.2% | 56.4 | 39.8 | 2.7 | 0.5 |
| 149.0 gms Aniline, 1.58 moles, 8:1 vs NB | 100.0% | 97.9% | 73.8 | 24.1 | 1.8 | 0.3 |

| TEMPERATURE | | | | | | |
|---|---|---|---|---|---|---|
| 70°C | 100.0% | 98.1 % | 65.1 | 33.0 | 1.4 | 0.5 |
| 90°C | 100.0% | 97.1 % | 71.9 | 25.1 | 2.7 | 0.2 |

| NITROBENZENE FEED RATE: | | | | | | |
|---|---|---|---|---|---|---|
| 29 minutes | 100.0% | 85.2% | 62.7 | 22.5 | 14.4 | 0.4 |
| 86 minutes BASE CHARGE: | 99.9% | 96.1% | 76.8 | 19.2 | 3.5 | 0.4 |
| 18.7 g 45% KOH, 0.15 mol, 0.75:1 vs NB | 83.0% | 97.6% | 63.1 | 17.9 | 1.8 | 0.2 |
| 37.4 g 45% KOH, 0.30 mol, 1.5:1 vs NB | 100.0% | 96.9% | 72.7 | 24.2 | 2.7 | 0.4 |

| ATMOSPHERE: | | | | | | |
|---|---|---|---|---|---|---|
| Vacuum, No Air | 100.0% | 85.5% | 66.5 | 19.0 | 14.2 | 0.3 |

| | | | | | | |
|---|---|---|---|---|---|---|
| *6:1 Aniline/NB, 80°C, 49 min. NB feed time, 1.25 moles KOH vs NB, air atmosphere | | | | | | |

### EXAMPLE 12

This example demonstrates the reaction of aniline and nitrobenzene in the presence of an oxidant in combination with an aqueous solution of tetramethylammonium hydroxide and various inorganic salts by continuous distillation of the aniline-water azeotrope. The TMAH/salt combination represents an ionic mixture of a potential base recycle stream from a process comprising an inorganic base and phase transfer catalyst after reduction of the coupling reaction mass to 4-ADPA.

Charged to a 500-mL round bottom flask equipped with a Teflon paddle stirrer, thermocouple, nitrobenzene feed tube and air bleed valve were: 139.7 grams aniline (99%, 1.49 moles), 73.9 grams aqueous tetramethylammonium hydroxide solution (35.5%, 0.29 moles) and an equivalent amount of salt (vs. base, in 15% molar excess over nitrobenzene) as listed in Table 18 below. The mixture was heated for 30 minutes at 16 kPa (120 mm Hg) and then nitrobenzene feed (30.8 grams, 99%, 0.25 moles) was started. The system pressure was regulated by adjusting the air bleed valve throughout the duration of the reaction cycle to maintain the desired temperature of 80°C and to complete the NB charge in approximately 75 minutes at a final pressure of 9.6 kPa (72 mm Hg). The mixture was held for 30 minutes at 9.3 kPa (70 mm Hg) to insure completeness of reaction and then quenched with 25 mL water. Air was bled into the reactor headspace during the entire cycle of charging reactants, heating to reaction temperature, feeding nitrobenzene and holding for reaction completion. The salts are charged in molar equivalence to nitrobenzene at Salt/NB =1.15. For example, potassium carbonate and sodium sulfate have two equivalents of inorganic cation, so that the molar ratio is 0.575.

The results with KCI at a slightly lower mole ratio to nitrobenzene agree well with results obtained from the use of strong base and phase transfer catalyst (KOH and TMACI), with continuous distillation of the aniline-water azeotrope. This demonstrates that use of an inorganic salt and organic base is equivalent to use of a strong base and phase transfer catalyst. It may be noted that sodium is not as effective as potassium for completing the reaction. Nitrate and bromide are also less effective anions for reaction completion at the conditions of this example. However, it should be possible to increase conversion for these salts by modifying reaction conditions, such as increasing reaction temperature. Most significant is the positive effect of salt addition on reaction selectivity. Comparison of the second and third experiments in Table 18 above shows that with the addition of KCI only, azobenzene was reduced by nearly two-thirds and relatively small amounts of "Other" compounds such as 4-Phenazo-diphenylamine were formed. The "TMAH Only" run was also characterized by high levels of compounds such as N-methylaniline and a stench of trimethylamine, both of which are indicative of base degradation.

**Table 18**

| | Conversion | Selectivity | Yield, % | | | |
|---|---|---|---|---|---|---|
| | % | % | *p*-NDPA | 4-NDPA | An Recr | Other |
| Comparison: KOH + TMACI, * | 100.0 | 97.9 | 72.6 | 25.3 | 1.9 | 0.2 |
| TMAH Only, No Salt Added | 100.0 | 83.8 | 62.4 | 21.4 | 4.4 | 11.8 |
| 21.44 g Potassium Chloride | 100.0 | 97.2 | 72.3 | 24.8 | 1.5 | 1.4 |
| 16.82 g Sodium Chloride | 62.7 | 97.2 | 15.6 | 45.3 | 0.6 | 1.1 |
| 19.87 g Potassium Carbonate | 100.0 | 89.1 | 72.8 | 16.3 | 3.8 | 7.1 |
| 20.42 g Sodium Sulfate | 100.0 | 85.0 | 63.8 | 21.2 | 4.5 | 10.5 |
| 24.44 g Sodium Nitrate | 27.8 | 94.3 | 8.3 | 17.9 | 0.9 | 0.6 |
| 34.22 g Potassium Bromide | 27.0 | 98.4 | 15.6 | 11.0 | 0.3 | 0.2 |
| 23.58 g Sodium Acetate | 80.5 | 96.9 | 56.6 | 21.4 | 1.5 | 1.0 |
| 19.55 g Sodium Formate | 71.8 | 97.3 | 46.2 | 23.6 | 1.0 | 1.0 |
| 24.18 g Potassium Formate | 89.5 | 96.4 | 64.2 | 22.1 | 2.4 | 0.8 |
| 39.13 g KH₂PO₄ | 39.3 | 97.4 | 10.0 | 28.3 | 0.8 | 0.2 |
| * Mole ratios are slightly higher: KOH/NB and TMACI/NB = 1.25 | | | | | | |

### EXAMPLE 13

This example demonstrates the effect of the mole ratio of inorganic salt to nitrobenzene. Reaction conditions were comparable to those for Example 12, except that the mole ratio of KCI to nitrobenzene was varied. The results in Table 19 indicate the addition of only a small amount of inorganic salt will increase selectivity. Therefore, in situations where corrosion due to high salt level is a concern, at least a modest selectivity improvement can be obtained.

**Table 19**

| | Mole Ratio | Conversion | Selectivity | Yield, % | | | |
|---|---|---|---|---|---|---|---|
| | Salt/NB | % | % | *p*-NDPA | 4-NDPA | An Recr | Other |
| TMAH Only, No Salt Added | 0 | 100.0 | 83.8 | 62.4 | 21.4 | 4.4 | 11.8 |
| 4.66 g KCl | 0.25 | 100.0 | 87.1 | 63.4 | 23.7 | 7.8 | 5.1 |
| 13.05 g KCl | 0.70 | 100.0 | 93.8 | 72.3 | 21.5 | 5.0 | 1.2 |
| 21.44 g KCl | 1.15 | 100.0 | 97.2 | 72.3 | 24.8 | 1.5 | 1.4 |

### EXAMPLE 14

This example demonstrates the effect of adding a non-salt compound on selectivity and conversion of nitrobenzene. Reaction conditions were comparable to those for Example 12. Betaine, i.e. (acetyl)trimethylammonium hydroxide inner salt, is a salt formed by the acetate group with the positively charged tetraalkylammonium group. So despite the name, the compound does not actually have hydroxide associated with the tetraalkylammonium group.
However, when a strong base is added, betaine is converted to a compound that contains both an acetate salt group and a tetraalkylammonium hydroxide group. So with TMAH, betaine is converted to a compound with a tetramethylammonium-acetate group and an (acetyl)trimethylammonium hydroxide group. With KOH, the compound has a potassium -acetate group with the (acetyl)trimethylammonium hydroxide group. In the KOH case, the compound represents a metal organic salt and a organic base in one molecule. Betaine is known in the literature to be a phase transfer compound or PTC (Starks and Liotta, ibid), as it carries the inorganic or organic base into the organic phase.

The results in Table 20 show that betaine has only a modest effect on selectivity or conversion with TMAH. The results with betaine/NB =1.15 are only equivalent to KCI/NB = 0.25. Furthermore, addition of an anion without an inorganic cation (ammonium acetate) is essentially ineffective. Therefore, the use of an inorganic salt or metal organic salt is the key to best results.

**Table 20**

| | | Conversion | Selectivity | Yield, % | | | |
|---|---|---|---|---|---|---|---|
| | Mole Ratio to NB | % | % | *p*-NDPA | 4-NDPA | An Recr | Other |
| TMAH Only, No Salt or PTC Added | 0 | 100.0 | 83.8 | 62.4 | 21.4 | 4.4 | 11.8 |
| 4.66 g KCl | 0.25 | 100.0 | 87.1 | 63.4 | 23.7 | 7.8 | 5.1 |
| 21.44 g KCl | 1.15 | 100.0 | 97.2 | 72.3 | 24.8 | 1.5 | 1.4 |
| 22.16 g Ammonium Acetate | 1.15 | 0.5 | 100.0 | 0.3 | 0.2 | 0.0 | 0.0 |
| 33.68 g Betaine | 1.15 | 100.0 | 87.6 | 70.7 | 16.9 | 4.8 | 7.6 |

### EXAMPLE 15

This example illustrates that use of partial oxidative conditions can give a significant increase of selectivity. Reaction conditions were comparable to those for Example 12, except as indicated. The results are shown in Table 21. Reaction 1 had comparable conditions to those for Example 12 throughout. For Reaction 2, the air bleed was used only during nitrobenzene feed and was stopped when 75% of the feed was completed. For Reaction 3, the nitrobenzene feed time was shortened to 45 minutes and the hold time was increased to 60 minutes, while the air bleed was used only during the nitrobenzene feed time. It is expected that higher selectivity will also be attained for sulfate, carbonate and nitrate by use of partial oxidative conditions.

**Table 21**

| | Conversion | Selectivity | Yield, % | | | |
|---|---|---|---|---|---|---|
| | % | % | *p*-NDPA | 4-NDPA | An Recr | Other |
| 16.70 g KF-1 | 99.9 | 84.8 | 67.3 | 17.4 | 4.2 | 11.0 |
| 16.70 g KF-2 | 100.0 | 91.1 | 80.1 | 11.0 | 6.3 | 2.6 |
| 16.70 g KF-3 | 100.0 | 93.8 | 83.2 | 10.6 | 4.3 | 1.9 |

### EXAMPLE 16 (comparative)

This example shows that use of an oxidant with a strong base, which also functions as a phase transfer catalyst, can increase selectivity. The reactions were done with azeotropic removal of water and aniline.

Charged to a 500-mL round bottom flask equipped with a Teflon paddle stirrer, thermocouple, nitrobenzene feed tube and air bleed valve were: 145.28 grams aniline (1.56 moles) and 87.36 grams aqueous tetramethylammonium hydroxide solution (36.0%, 0.345 moles) for Runs 1 -3. The mixture was heated for 30 minutes at 16 kPa (120 mm Hg) and then nitrobenzene feed (36.93 grams, 0.30 moles) was started. The system pressure was held constant at 9.33 kPa (70 mm Hg) throughout the reaction period. Temperature rose from about 66°C to about 80°C during the reaction period. Nitrobenzene was charged over about 80 minutes, after which the batch was held for 40 minutes at 9.33 kPa (70 mm Hg) to insure completeness of reaction and then quenched with 25 mL water. Hydrogen peroxide was charged as 20.40 grams (0.03 moles) of a 5 wt.% aqueous solution concurrently with nitrobenzene. Since water can also affect selectivity, by protecting TMAH from degradation and shifting reaction equilibria, a control was run with 20.40 grams of water that was also fed concurrently with nitrobenzene. Runs 4 - 7 had slightly different conditions. The main difference was starting with 25 wt.% TMAH with removal of water and some aniline in the reactor prior to the nitrobenzene feed. Air was added either during the entire nitrobenzene feed or for half of the feed time.

The results in Table 22 show that hydrogen peroxide gives a larger selectivity improvement than water alone, showing that use of an oxidant can be beneficial. The results also show that addition of air as oxidant over the entire reactor cycle has a deleterious effect on selectivity. However, a selectivity improvement is obtained when the air addition is limited to part of the reaction cycle, showing that partial oxidative conditions can be beneficial. These reactions were run somewhat wetter than the case for 100% air in Table 18, which explains the lower selectivity in Table 18. Runs 4 and 5 show the excellent repeatability of the reactions, so that selectivity increases of 1 - 2% are significant.

**Table 22**

| | Conversion | Selectivity | Yield, % | | | |
|---|---|---|---|---|---|---|
| TMAH as base/PTC | % | % | *p*-NDPA | 4-NDPA | An Recr | Other |
| 1. No oxidant | 100.0 | 93.3 | 86.3 | 7.0 | 4.6 | 2.1 |
| 2. Water only | 100.0 | 94.9 | 89.3 | 5.5 | 3.4 | 1.8 |
| 3. Hydrogen Peroxide | 100.0 | 96.1 | 89.7 | 6.4 | 1.9 | 2.0 |
| 4. No oxidant | 100.0 | 92.3 | 86.5 | 5.8 | 5.9 | 1.8 |
| 5. No oxidant | 100.0 | 92.2 | 86.2 | 6.0 | 6.1 | 1.7 |
| 6. Air, 100% NB Feed | 99.7 | 89.4 | 79.0 | 10.1 | 7.4 | 3.2 |
| 7. Air, 50% NB Feed | 100.0 | 94.5 | 88.4 | 6.2 | 4.2 | 1.3 |

## Claims

1. A method of producing 4-aminodiphenylamine or substituted derivatives thereof comprising the steps of:
(a) bringing an aniline or aniline derivative selected from the group consisting of formanilide, phenylurea, carbanilide and thiocarbanilide, or from the group consisting of 2-methoxyaniline, 4-methoxyaniline, 4-chloroaniline, p-toluidine, 4-nitroaniline, 3-bromoaniline, 3-bromo-4-aminotoluene, p-aminobenzoic acid, 2, 4-diaminotoluene, 2,5-dichloroaniline, 1,4-phenylene diamine, 4,4'-methylene dianiline, 1,3,5-triaminobenzene, and mixtures thereof, into reactive contact with nitrobenzene;
(b) obtaining a 4-aminodiphenylamine intermediate product by reacting the aniline or aniline derivative and nitrobenzene in a confined zone at a suitable time, pressure and temperature in the presence of a mixture comprising an organic base, and an inorganic salt or a metal organic salt, or mixtures thereof, selected from the group consisting of fluoride, chloride, bromide, sulfate, hydrogen sulfate, nitrate, phosphate, dihydrogen phosphate, formate, acetate, oxalate, malonate, citrate, tartrate, maleate, chlorate, perchlorate, chromate, rhenate and carbonate salts of cesium, rubidium, potassium and sodium, and mixtures thereof, and an oxidant, wherein the organic base is selected from the group consisting of tetramethylammonium hydroxide, tetrabutylammonium hydroxide, methyltributylammonium hydroxide, benzyltrimethylammonium hydroxide, tricaprylmethylammonium hydroxide, cetyltrimethylammonium hydroxide, and choline hydroxide; and
(c) reducing the 4-aminodiphenylamine intermediate product of step (b) to produce 4-aminodiphenylamine or substituted derivatives thereof.

2. The method of claim 1 wherein the molar ratio of organic base to nitrobenzene is greater than or equal to 1 : 1.

3. The method of claim 1 wherein the molar ratio of organic base to nitrobenzene is greater than or equal to 1.1 : 1 to 6 : 1.

4. The method of claim 1 wherein the molar ratio of inorganic salt or metal organic salt to nitrobenzene is from 0.05 : 1 to 6.5 : 1.

5. The method of claim 1 wherein said oxidant is hydrogen peroxide.

6. The method of claim 1 wherein said oxidant is free oxygen.

7. The method of claim 1 wherein said oxidant is present only for part of the time during which the aniline and nitrobenzene react.

8. The method of claim 1 wherein said reactive contact is carried out at a temperature of from 20 °C to 150 °C, a pressure in the range of from 20 mbar to 20 barg and a reaction time less than 3.5 hours.

9. The method of claim 1 wherein the reaction of step (b) is carried out in the presence of not greater than 10 : 1 moles water to moles nitrobenzene excluding water of hydration.

10. The method of claim 1 wherein said mixture comprising an organic base and an inorganic salt or metal organic salt is in aqueous solution and the reaction is carried out with a continuous distillation of aniline-water azeotrope, optionally wherein the reaction mixture is agitated during the entire reaction.

11. The method of claim 1 wherein said reactive contact occurs in a solvent system.

12. The method of claim 11 wherein said solvent system comprises a polar aprotic solvent.

13. The method of claim 12 wherein said polar aprotic solvent is selected from the group consisting of dimethyl sulfoxide, benzyl ether, 1-methyl-2 pyrrolidinone, and N,N-dimethylformamide.

14. The method of claim 1 wherein the 4-aminodiphenylamine produced is reductively alkylated to an alkylated derivative of the 4-aminodiphenylamine.

## Patentansprüche

1. Ein Verfahren zur Herstellung von 4-Aminodiphenylamin oder eines substituierten Derivates davon, das die Schritte umfasst:
(a) reaktives in Kontakt bringen von Anilin oder eines Derivates davon ausgewählt aus der Gruppe bestehend aus Formanilid, Phenylharnstoff, Carbanilid und Thiocarbanilid, oder aus der Gruppe bestehend aus 2-Methoxyanilin, 4-Methoxyanilin, 4-Chloroanilin, p-Toluidin, 4-Nitroanilin, 3-Bromanilin, 3-Brom-4-Aminotoluol, p-Aminobenzoesäure, 2,4-Diaminotoluol, 2,5-Dichloranilin, 1,4-Phenyldiamin, 4,4'-Methylenanilin, 1,3,5-Triaminobenzol und Mischungen davon, mit Nitrobenzol;
(b) Erhalten eines 4-Aminodiphenylamin Zwischenprodukts durch das Umsetzen des Anilins oder Anilinderivates mit Nitrobenzol in einer begrenzten Zone über eine geeignete Zeit, bei einem geeigneten Druck und bei einer geeigneten Temperatur in Gegenwart eines Gemischs, das eine organische Base und ein anorganisches Salz oder ein metallorganisches Salz oder Gemische davon ausgewählt aus der Gruppe bestehend aus Fluorid, Chlorid, Bromid, Sulfat, Hydrogensulfat, Nitrat, Phosphat, Dihydrogenphosphat, Formiat, Acetat, Oxalat, Malonat, Citrat, Tartrat, Maleat, Chlorat, Perchlorat, Chromat, Rhenat und Carbonatsalzen des Cäsiums, Rubidiums, Kaliums und Natriums und Gemische davon und ein Oxidationsmittel enthält, wobei die organische Base ausgewählt ist aus der Gruppe bestehend aus Tetramethylammoniumhydroxid, Tetrabutylammoniumhydroxid, Methyltributyl-ammoniumhydroxid, Benzyltrimethylammoniumhydroxid, Tricaprylmethyl-ammoniumhydroxid, Cetyltrimethylammoniumhydroxid und Cholinhydroxid; und
(c) Reduzieren des 4-Aminodiphenylamin Zwischenprodukts des Schritts (b) um 4-Aminodiphenylamin oder substituierte Derivate davon herzustellen.

2. Das Verfahren nach Anspruch 1, wobei das molare Verhältnis der organischen Base zu Nitrobenzol größer oder gleich 1 : 1 ist.

3. Das Verfahren nach Anspruch 1, wobei das molare Verhältnis der organischen Base zu Nitrobenzol größer oder gleich 1,1 : 1 bis 6 : 1 ist.

4. Das Verfahren nach Anspruch 1, wobei das molare Verhältnis des anorganischen Salzes oder metallorganischen Salzes zu Nitrobenzol von 0,05 : 1 bis 6,5 : 1 ist.

5. Das Verfahren nach Anspruch 1, wobei das Oxidationsmittel Wasserstoffperoxid ist.

6. Das Verfahren nach Anspruch 1, wobei das Oxidationsmittel freier Sauerstoff ist.

7. Das Verfahren nach Anspruch 1, wobei das Oxidationsmittel nur für einen Teil der Zeit während der Anilin und Nitrobenzol reagieren zugegen ist.

8. Das Verfahren nach Anspruch 1, wobei das reaktive in Kontakt bringen bei einer Temperatur von 20 °C bis 150 °C, bei einem Druck im Bereich von 20 mbar bis 20 barg und einer Reaktionszeit von weniger als 3,5 Stunden durchgeführt wird.

9. Das Verfahren nach Anspruch 1, wobei der Schritt (b) in Gegenwart von nicht mehr als 10 : 1 Mol Wasser zu Mol Nitrobenzol, nicht eingerechnet das Wasser der Hydratisierung, durchgeführt wird.

10. Das Verfahren nach Anspruch 1, wobei das Gemisch, das eine organische Base und ein anorganisches Salz oder metallorganisches Salz umfasst, in einer wässrigen Lösung ist und die Reaktion mittels kontinuierlicher Destillation eines Anilin-Wasser-Azeotrops durchgeführt wird, wobei das Reaktionsgemisch während der gesamten Reaktion optional gerührt wird.

11. Das Verfahren nach Anspruch 1, wobei der reaktive Kontakt in einem Lösungsmittelsystem erfolgt.

12. Das Verfahren nach Anspruch 11, wobei das Lösungsmittelsystem ein polar aprotisches Lösungsmittel umfasst.

13. Das Verfahren nach Anspruch 12, wobei das polar aprotische Lösungsmittel ausgewählt ist aus der Gruppe bestehend aus Dimethylsulfoxid, Benzylether, 1-Methyl-2-Pyrrolidon und N,N-Dimethylformamid.

14. Das Verfahren nach Anspruch 1, wobei das hergestellte 4-Aminodiphenylamin reduktiv zu einem alkylierten Derivat des 4-Aminodiphenylamins alkyliert wird.

## Revendications

1. Procédé de production d'une 4-aminodiphénylamine ou de l'un de ses dérivés substitués, comprenant les étapes suivantes :
(a) la mise en contact réactif d'une aniline ou d'un dérivé d'aniline choisi dans le groupe constitué du formanilide, de la phénylurée, du carbanilide et du thiocarbanilide, ou dans le groupe constitué de la 2-méthoxyaniline, de la 4-méthoxy-aniline, de la 4-chloroaniline, de la p-toluidine, de la 4-nitroaniline, de la 3-bromo-aniline, du 3-bromo-4-aminotoluène, de l'acide p-aminobenzoïque, du 2,4-diamino-toluène, de la 2,5-dichloroaniline, de la 1,4-phénylène diamine, de la 4,4'-méthylène dianiline, du 1,3,5-triaminobenzène, et de leurs mélanges, avec du nitrobenzène ;
(b) l'obtention d'un produit intermédiaire de 4-aminodiphénylamine par la réaction de l'aniline ou du dérivé d'aniline et du nitrobenzène dans une zone confinée pendant une durée appropriée, sous une pression appropriée et à une température appropriée en présence d'un mélange comprenant une base organique, et un sel inorganique ou un sel métallique organique, ou leurs mélanges, choisi dans le groupe constitué des sels de fluorure, de chlorure, de bromure, de sulfate, d'hydrogénosulfate, de nitrate, de phosphate, de dihydrogénophosphate, de formiate, d'acétate, d'oxalate, de malonate, de citrate, de tartrate, de maléate, de chlorate, de perchlorate, de chromate, de rhénate et de carbonate de césium, de rubidium, de potassium et de sodium, et leurs mélanges, et un oxydant, dans lequel la base organique est choisie dans le groupe constitué de l'hydroxyde de tétraméthylammonium, de l'hydroxyde de tétrabutylammonium, de l'hydroxyde de méthyltributylammonium, de l'hydroxyde de benzyltriméthylammonium, de l'hydroxyde de tricaprylméthylammonium, de l'hydroxyde de cétyltriméthylammonium et de l'hydroxyde de choline ; et
(c) la réduction du produit intermédiaire de 4-aminodiphénylamine de l'étape (b) pour produire une 4-aminodiphénylamine ou un de ses dérivés substitués.

2. Procédé selon la revendication 1, dans lequel le rapport molaire entre la base organique et le nitrobenzène est supérieur ou égal à 1 : 1.

3. Procédé selon la revendication 1, dans lequel le rapport molaire entre la base organique et le nitrobenzène est supérieur ou égal à 1,1 : 1 à 6 : 1.

4. Procédé selon la revendication 1, dans lequel le rapport molaire entre le sel inorganique ou le sel métallique organique et le nitrobenzène est de 0,05 : 1 à 6,5 : 1.

5. Procédé selon la revendication 1, dans lequel ledit oxydant est le peroxyde d'hydrogène.

6. Procédé selon la revendication 1, dans lequel ledit oxydant est l'oxygène libre.

7. Procédé selon la revendication 1, dans lequel ledit oxydant est présent seulement pendant une partie de la durée pendant laquelle l'aniline et le nitrobenzène réagissent.

8. Procédé selon la revendication 1, dans lequel ledit contact réactif est réalisé à une température de 20 °C à 150 °C, sous une pression située dans la plage allant de 20 mbar à 20 barg et pendant une durée de réaction inférieure à 3,5 heures.

9. Procédé selon la revendication 1, dans lequel la réaction de l'étape (b) est réalisée en présence d'au plus 10 : 1 mole d'eau par mole de nitrobenzène en excluant l'eau d'hydratation.

10. Procédé selon la revendication 1, dans lequel ledit mélange comprenant une base organique et un sel inorganique ou un sel métallique organique est dans une solution aqueuse et la réaction est réalisée avec une distillation continue de l'azéotrope aniline-eau, facultativement dans lequel le mélange réactionnel est agité pendant toute la réaction.

11. Procédé selon la revendication 1, dans lequel ledit contact réactif a lieu dans un système de solvants.

12. Procédé selon la revendication 11, dans lequel ledit système de solvants comprend un solvant aprotique polaire.

13. Procédé selon la revendication 12, dans lequel ledit solvant aprotique polaire est choisi dans le groupe constitué du diméthylsulfoxyde, du benzyléther, de la 1méthyl-2-pyrrolidinone et du N,N-diméthylformamide.

14. Procédé selon la revendication 1, dans lequel la 4-aminodiphénylamine produite est alkylée en conditions réductrices pour donner un dérivé alkylé de la 4-amino-diphénylamine.
